# EUROPEAN PATENT SPECIFICATION

(11) **EP 2 258 389 B1**
(45) Date of publication and mention of the grant of the patent: **05.06.2013**
(21) Application number: 10179783.5
(22) Date of filing: 01.09.2003
(51) Int. Cl.: A61K 39/095, A61K 39/104, A61K 39/112, A61K 9/00

(54) **Improved bacterial outer membrane vesicles**
Verbesserte Vesikel auf äußeren Membranen von Bakterien
Vésicules de membrane externe bactérienne améliorées

(30) Priority: 30.08.2002 GB 0220194
(43) Date of publication of application: 08.12.2010
(62) Divisional of application: 07012893.9
(73) Proprietor: Novartis Vaccines and Diagnostics S.r.l., 53100 Siena (SI) (IT)
(72) Inventor: Pizza, Mariagrazia, 53100 Siena (IT); Serruto, Davide, 53100 Siena (IT); Rappuoli, Rino, I-53100 Siena (IT)
(74) Representative: Marshall, Cameron John

(56) References cited:
- WO-A-01/91788
- WO-A-02/062378
- US-A1- 2002 110 569

## Description

### TECHNICAL FIELD

This invention is in the field of vesicle preparation for immunisation purposes.

### BACKGROUND ART

One of the various approaches to immunising against *N.meningitidis* infection is to use outer membrane vesicles (OMVs). An efficacious OMV vaccine against serogroup B has been produced by the Norwegian National Institute of Public Health [*e.g*. ref. 1] but, although this vaccine is safe and prevents MenB disease, its efficacy is limited to the strain used to make the vaccine.

The 'RIVM' vaccine is based on vesicles containing six different PorA subtypes and has been shown to be immunogenic in children in phase II clinical trials [2].

Reference 3 discloses a vaccine against different pathogenic serotypes of serogroup B meningococcus based on OMVs which retain a protein complex of 65-kDa. Reference 4 discloses a vaccine comprising OMVs from genetically-engineered meningococcal strains, with the OMVs comprising: at least one Class 1 outer-membrane protein (OMP) but not comprising a Class 2/3 OMP. Reference 5 discloses OMVs comprising OMPs which have mutations in their surface loops and OMVs comprising derivatives of meningococcal lipopolysaccharide (LPS).

Reference 6 discloses compositions comprising OMVs supplemented with transferrin binding proteins (*e.g.* TbpA and TbpB) and/or Cu,Zn-superoxide dismutase. Reference 7 discloses compositions comprising OMVs supplemented by various proteins. Reference 8 discloses preparations of membrane vesicles obtained from *N.meningitidis* with a modified *fur* gene.

As well as serogroup B *N.meningitidis,* vesicles have been prepared for other bacteria. Reference 9 discloses a process for preparing OMV-based vaccines for serogroup A meningococcus. References 10 and 11 disclose vesicles from *N.gonorrhoeae.* Reference 12 discloses vesicle preparations from *N.lactamica.* Vesicles have also been prepared from *Moraxella catarrhalis* [13,14], *Shigella flexneri* [15,16], *Pseudomonas aeruginosa* [15,16], *Porphyromonas gingivalis* [17], *Treponema pallidum* [18], *Haemophilus influenzae* [19 & 20] and *He*/*icobacter pylori* [21].

One drawback with bacterial vesicle preparations is that important protective antigens are not present. To retain antigens such as NspA in OMV preparations, reference 20 teaches that *nspA* expression should be up-regulated with concomitant *porA* and *cps* knockout. It is an object of the invention to provide further and improved vesicle preparations, together with processes for their manufacture. In particular, it is an object of the invention to provide vesicles which retain important bacterial immunogenic components from *N.meningitidis.*

### DISCLOSURE OF THE INVENTION

The invention provides a composition comprising meningococcal outer membrane vesicles, an aluminium hydroxide adjuvant, a histidine buffer and sodium chloride, wherein: (a) the concentration of sodium chloride is greater than 7.5 mg/ml; and/or (b) the concentration of OMVs is less than 100µg/ml.

The concentration of sodium chloride is preferably greater than 8 mg/ml, and is more preferably about 9 mg/ml.

The concentration of OMVs is preferably less than 75 µg/ml *e.g*. about 50 µg/ml

The histidine buffer is preferably between pH 6.3 and pH 6.7 *e.g.* pH 6.5.

The adjuvant may be used at about 3.3 mg/ml (expressed as Al³⁺ concentration).

The bacterium from which OMVs are prepared is *N.meningitidis*. Within *N.meningitidis*, any of serogroups A, C, W135 and Y may be used, but it is preferred to prepare vesicles from serogroup B. Preferred strains within serogroup B are MC58, 2996, H4476 and 394/98.

To reduce pyrogenic activity, it is preferred that the bacterium should have low endotoxin (LPS) levels. Suitable mutant bacteria are known *e.g.* mutant *Neisseria* [23]. Processes for preparing LPS-depleted outer membranes from Gram-negative bacteria are disclosed in reference 25

The bacterium may be a wild-type bacterium, or it may be a recombinant bacterium. Preferred recombinant bacteria over-express (relative to the corresponding wild-type strain) immunogens such as NspA, 287, 741, TbpA, TbpB, superoxide dismutase [6], *etc.* The bacterium may express more than one PorA class I outer membrane protein *e.g.* 2, 3, 4, 5 or 6 of PorA subtypes: P1.7,16; P1.5,2; P1.19,15; P1.5c,10;P1.12,13; and P1.7h,4 [*e.g*. refs. 26, 27].

### The vesicles

OMVs are prepared from the outer membrane of cultured bacteria. They may be obtained from bacteria grown in broth or in solid medium culture, preferably by separating the bacterial cells from the culture medium (*e.g.* by filtration or by a low-speed centrifugation to pellet the cells), lysing the cells (without detergent), and separating an outer membrane fraction from cytoplasmic molecules (*e.g.* by filtration, by differential precipitation or aggregation of outer membranes and/or OMVs, by affinity separation methods using ligands that specifically recognize outer membrane molecules, or by a high-speed centrifugation that pellets outer membranes and/or OMVs).

OMVs can be distinguished from microvesicles (MVs [28]) and 'native OMVs' ('NOMVs' [66]), which arc naturally-occurring membrane vesicles that form spontaneously during bacterial growth and are released into culture medium. MVs can be obtained by culturing *Neisseria* in broth culture medium, separating whole cells from the broth culture medium (*e.g.* by filtration or by low-speed centrifugation to pellet only the cells and not the smaller blebs) and then collecting the MVs that are present in the cell-depleted medium (*e.g.* by filtration, by differential precipitation or aggregation of MVs, by high-speed centrifugation to pellet the MVs). Strains for use in production of MVs can generally be selected on the basis of the amount of MVs produced in culture. References 29 and 30 describe *Neisseria* with high MV production.

### Immunogenic pharmaceutical compositions

For administration to a patient, the vesicles are formulated as immunogenic compositions, and more preferably as compositions suitable for use as a vaccine in humans (*e.g.* children or adults). Vaccines of the invention may either be prophylactic (*i.e.* to prevent infection) or therapeutic (*i.e.* to treat disease after infection), but will typically be prophylactic.

The composition of the invention is preferably sterile.

The composition of the invention is preferably pyrogen-free.

The composition of the invention generally has a pH of between 6.0 and 7.0, more preferably to between 6.3 and 6.9 *e.g.* 6.6±0.2. The composition is preferably buffered at this pH.

Other components suitable for human administration arc disclosed in reference 44.

The composition comprises an adjuvant. The adjuvant is aluminium hydroxide (including oxyhydroxides).

The vesicles in the compositions of the invention will be present in 'immunologically effective amounts' *i.e.* the administration of that amount to an individual, either in a single dose or as part of a series, is effective for treatment or prevention of disease. This amount varies depending upon the health and physical condition of the individual to be treated, age, the taxonomic group of individual to be treated (*e.g.* non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g.* including booster doses). The vaccine may be administered in conjunction with other immunoregulatory agents.

Typically, the compositions of the invention are prepared as injectables. Direct delivery of the compositions will generally be parenteral (*e.g.* by injection, either subcutaneously, intraperitoneally, intravenously or intramuscularly or delivered to the interstitial space of a tissue) or mucosal (*e.g.* oral or intranasal [65,66]). The compositions can also be administered into a lesion.

Once formulated, the compositions of the invention can be administered directly to the subject. The subjects to be treated can be animals; in particular, human subjects can be treated. The vaccines are particularly useful for vaccinating children and teenagers.

The composition may comprise vesicles from more than one serosubtype of *N.meningitidis* [28]. Similarly, the composition may comprise more than one type of vesicle *e.g.* both MVs and OMVs As well as vesicles, the composition of the invention may comprise further antigens. For example, the composition may comprise one or more of the following further antigens:
- antigens from *Helicobacter pylori* such as CagA [67 to 70], VacA [71, 72], NAP [73, 74, 75], HopX [*e.g.* 76], HopY [*e.g.* 76] and/or urease.
- a saccharide antigen from *N.meningitidis* serogroup A, C, W135 and/or Y, such as the oligosaccharide disclosed in ref. 77 from serogroup C [see also ref. 78] or the oligosaccharides of ref. 79.
- a saccharide antigen from *Streptococcus pneumoniae* [*e.g.* 80, 81,82].
- an antigen from hepatitis A virus, such as inactivated virus [*e.g*. 83, 84].
- an antigen from hepatitis B virus, such as the surface and/or core antigens [*e.g.* 84, 85].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g*. refs. 86 & 87].
- a diphtheria antigen, such as a diphtheria toxoid *[e.g.* chapter 3 of ref. 88] *e.g.* the CRM₁₉₇ mutant [*e.g*. 89].
- a tetanus antigen, such as a tetanus toxoid [*e.g*. chapter 4 of ref. 108].
- a saccharide antigen from *Haemophilus influenzae* B [*e.g.* 78].
- an antigen from hepatitis C virus [*e.g*. 90].
- an antigen from *N.gonorrhoeae* [*e.g*. 91, 92, 93, 94].
- an antigen from *Chlamydia pneumoniae* [*e.g.* refs. 95 to 101].
- an antigen from *Chlamydia trachomatis* [*e.g.* 102].
- an antigen from *Porphyromonas gingivalis* [*e.g.* 103].
- polio antigen(s) [*e.g*. 104, 105] such as OPV or, preferably, IPV.
- rabies antigen(s) [*e.g*. 106] such as lyophilised inactivated virus [*e.g*. 107, RabAvert™].
- measles, mumps and/or rubella antigens [*e.g.* chapters 9, 10 & 11 of ref. 108].
- influenza antigen(s) [*e.g*. chapter 19 of ref. 108], such as the haemagglutinin and/or neuraminidase surface proteins.
- an antigen from *Moraxella catarrhalis* [*e.g.* 109].
- an protein antigen from *Streptococcus agalactiae* (group B streptococcus) [*e.g*. 110, 111].
- a saccharide antigen from *Streptococcus agalactiae* (group B streptococcus).
- an antigen from *Streptococcus pyogenes* (group A streptococcus) [*e.g*. 111, 112, 113].
- an antigen from *Staphylococcus aureus* [*e.g.* 114].
- an antigen from *Bacillus anthracis* [*e.g.* 115, 116, 117].
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen *e.g.* from parvovirus B 19.
- a prion protein (*e.g.* the CJD prion protein)
- an amyloid protein, such as a beta peptide [118]
- a cancer antigen, such as those listed in Table 1 of ref. 119 or in tables 3 & 4 of ref. 120.

The composition may comprise one or more of these further antigens.

Toxic protein antigens may be detoxified where necessary (*e.g.* detoxification of pertussis toxin by chemical and/or genetic means [87]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include the *N.meningitidis* outer membrane protein [121], synthetic peptides [122,123], heat shock proteins [124,125], pertussis proteins [126,127], protein D from *H.influenzae* [128], cytokines [129], lymphokines [129], hormones [129], growth factors [129], toxin A or B from *C.difficile* [130], iron-uptake proteins [131], *etc.* A preferred carrier protein is the CRM197 diphtheria toxoid [132].

*N.meningitidis* serogroup B antigens may also be added to the OMV compositions. In particular, a protein antigen such as disclosed in refs. 133 to 139 may be added.

Antigens in the composition will typically be present at a concentration of at least 1 µg/ml each. In general, the concentration of any given antigen will be sufficient to elicit an immune response against that antigen.

### Methods of treating patients

The invention provides compositions of the invention for use as medicaments.

Compositions of the invention can be used in a method of raising an immune response in a patient, comprising administering to a patient a composition of the invention. The immune response is preferably protective against meningococcal disease, and may comprise a humoral immune response and/or a cellular immune response. The patient is preferably a child.

The method may raise a booster response, in a patient that has already been primed against *N.meningitidis.* Subcutaneous and intranasal prime/boost regimes for OMVs are disclosed in ref. 65.

The invention also provides the use of a vesicle of the invention in the manufacture of a medicament for raising an immune response in an patient. The medicament is an immunogenic composition as defined above (*e.g*. a vaccine). The medicament is preferably for the prevention and/or treatment of a disease caused by a *Neisseria* (*e.g.* meningitis, septicaemia, gonorrhoea *etc*.).

Uses of the invention may involve administration of vesicles from more than one serosubtype of *N.meningitidis* [*e.g*. ref. 28].

The term "comprising" means "including" as well as "consisting" *e.g.* a composition "comprising" X may consist exclusively of X or may include something additional *e.g.* X + Y.

The term "about" in relation to a numerical value *x* means, for example, *x*±10%.

The word "substantially" does not exclude "completely" *e.g.* a composition which is "substantially free" from Y may be completely free from Y. Where necessary, the word "substantially" may be omitted from the definition of the invention.

### BRIEF DESCRIPTION OF DRAWINGS

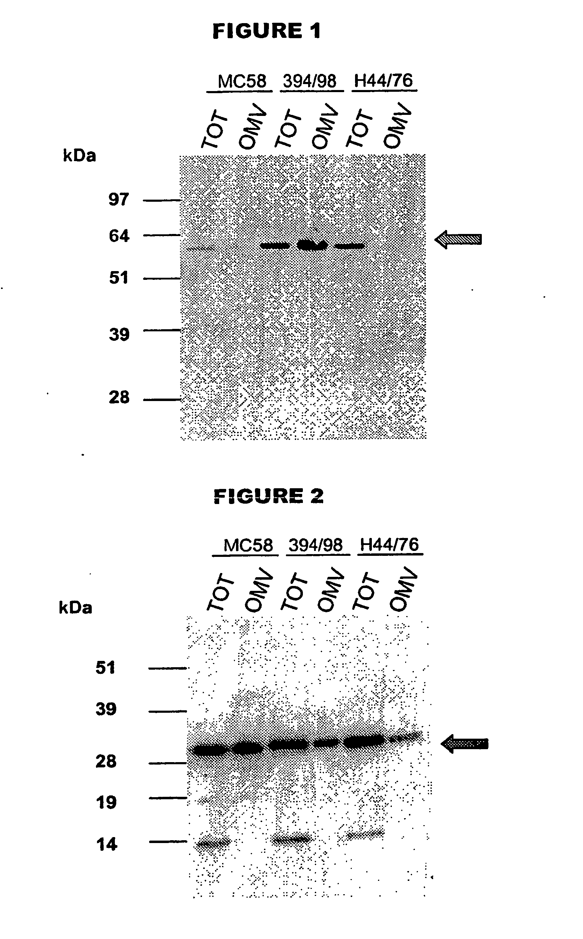

Figures 1 and 2 show the presence/absence of (1) protein '287' and (2) protein '741' in bacteria ('TOT') and outer membrane vesicles ('OMV') prepared from strains MC58, H4476 and 394/98 of *N.meningitidis.* The arrow shows the position of '287' in Figure 1 and '741' in Figure 2.

### MODES FOR CARRYING OUT THE INVENTION

### OMV preparation

OMVs were prepared either by the prior art 'Norwegian' methods (strains H4476 and 394/98) or by the following process (strain MC58):
- Bacteria from 2-5 plates were harvested into 10 ml of 10mM Tris-HCl buffer (pH 8.0) and heat-killed at 56°C for 45min. The samples were then sonicated on ice (duty cycle 50 for 10 minutes with the tip at 6/7) to disrupt membranes.
- Cellular debris was removed by centrifugation at 5000g for 30 minutes at 4°C, or 10000g for 10 minutes.
- The supernatant was re-centrifuged at 50000g for 75 minutes at 4°C
- The pellet was resuspended in 7 ml of 2% N-lauroyl sarcosinate (Sarkosyl) in 10 mM Tris-HCl (pH 8.0) for 20 minutes at room temperature to solubilise the cytoplasmic membranes.
- The sample was centrifuged at 10000g for 10 minutes to remove particulates and the supernatant was centrifuged at 75000g for 75 minutes at 4°C. The sample was washed in 10mM Tris-HCl (pH 8.0) and centrifuged at 75000g for 75 minutes.
- The pellet was resuspended in 10 mM Tris-HCl (pH 8.0) or distilled water.

The bacteria and the OMV preparations were tested by Western blot for the presence of NspA, 287 and 741 (Figures 1 & 2) and results are summarised in the following table:

| **Strain** | **Deterge t** | **NspA** | | **287** | | **741** | |
|---|---|---|---|---|---|---|---|
| | | **Bacteria** | **OMV** | **Bacteria** | **OMV** | **Bacteria** | **OMV** |
| MC58 | - | +++ | +++ | +++ | +++ | +++ | +++ |
| H44/76 | + | +++ | -^{[20]} | +++ | - | +++ | + |
| 394/98 | + | +++ | n.d. | +++ | ++ | +++ | + |

In contrast to the prior art detergent-based methods, therefore, the absence of detergent results in NspA being retained in OMVs and avoids loss of 287 & 741.

### Formulation of OMVs prepared from New Zealand strain of MenB

OMVs were prepared from serogroup B strain 394/98 of *N.meningitidis.* These were formulated in two different ways, with components having the following concentrations:

| | **Formulation 'A'** | **Formulation 'B'** |
|---|---|---|
| **OMVs** | 50 µg/ml | 50 µg/ml |
| **Aluminium hydroxide adjuvant** | 3.3 µg.ml | 3.3 µg.ml |
| **Sucrose** | 3% | - |
| **Histidine buffer, pH 6.5** | - | 5 mM |
| **Sodium chloride** | - | 9 mg/ml |

Formulation 'B' was found to be immunologically superior to formulation 'A'. Formulation 'B' differs from that disclosed in reference 142 by having half the OMV concentration a higher NaCl concentration, and a slightly different pH.

It will be understood that the invention has been described by way of example only and modifications may be made whilst remaining within the scope of the invention.
[1] Bjune et al. (1991) Lancet 338(8775):1093-1096.
[2] de Kleijn et al. (2001) Vaccine 20:352-358.
[3] US patents 5,597,572 & 5,747,653; see also European patent 0301992.
[4] European patent 0449958 (granted from WO90/06696).
[5] US patent 5,705,161; see also WO94/08021.
[6] International patent application WO00/25811.
[7] International patent application WO01/52885.
[8] International patent application WO98/56901.
[9] International patent application WO01/91788.
[10] Parmar et al. (1997) Vaccine 15:1641-1651.
[11] International patent application WO99/59625.
[12] International patent application WO00/50074.
[13] US patents 5,552,146, 5,981,213 & 5,993,826; see also WO93/03761.
[14] Zhou et al. (1998) FEMS Microbiol Lett 163:223-228.
[15] Kadurugamuwa & Beveridge (1999) Microbiology 145:2051-2060.
[16] International patent application WO97/05899.
[17] Kesavalu et al. (1992) Infect. Immun. 60:1455-1464.
[18] Blanco et al. (1999) J Immunol 163:2741-2746.
[19] International patent application WO01/09350.
[20] International patent application WO02/09746.
[21] Keenan et al. (1998) FEMS Microbiol Lett 161:21-27.
[22] European patent 0011243.
[23] International patent application WO99/10497.
[24] International patent application WO02/07763.
[25] European patent 0624376.
[26] Claassen et al. (1996) Vaccine 14:1001-1008.
[27] Peeters et al. (1996) Vaccine 14:1009-1015.
[28] International patent application WO02/09643.
[29] US patent 6,180,111.
[30] International patent application WO01/34642.
[31] Martin et al. (1997) J. Exp. Med. 185:1173-1183.
[32] Plante et al. (1999) Infect. Immun. 67:2855-2861.
[33] Cadieux et al. (1999) Infect. Immun. 67:4955-4959.
[34] Moe et al. (1999) Infect. Immun. 67:5664-5675.
[35] Martin et al. (2000) J. Biotechnol. 83:27-31.
[36] Moe et al. (2001) Infect. Immun. 69:3762-3771.
[37] International patent application WO96/29412.
[38] International patent application WO00/71725.
[39] Tettelin et al. (2000) Science 287:1809-1815.
[40] International patent application WO99/57280.
[41] International patent application WO03/020756 (SEQ IDs 1-22 in particular).
[42] International patent application WO00/66741.
[43] International patent application WO01/52885.
[44] Gennaro (2000) Remington: The Science and Practice of Pharmacy. 20th edition, ISBN: 0683306472.
[45] Vaccine design: the subunit and adjuvant approach, eds. Powell & Newman, Plenum Press 1995 (ISBN 0-306-44867-X).
[46] WO90/14837.
[47] WO02/26212.
[48] WO98/33487.
[49] WO00/07621.
[50] WO99/27960.
[51] WO98/57659.
[52] European patent applications 0835318, 0735898 and 0761231.
[53] Krieg (2000) Vaccine 19:618-622; Krieg (2001) Curr opin Mol Ther 2001 3:15-24; WO96/02555, WO98/16247, WO98/18810, WO98/40100, WO98/55495, WO98/37919 and WO98/52581 *etc.*
[54] WO99/52549.
[55] WO01/21207.
[56] WO01/21152.
[57] WO00/62800.
[58] WO00/23105.
[59] WO99/11241.
[60] Del Giudice et al. (1998) Molecular Aspects of Medicine, vol. 19, number 1.
[61] Vaccine Design... (1995) eds. Powell & Newman. ISBN: 030644867X. Plenum.
[62] Johnson et al. (1999) Bioorg Med Chem Lett 9:2273-2278.
[63] International patent application WO00/50078.
[64] Singh et al. (2001) J. Cont. Rele. 70:267-276.
[65] Bakke et al. (2001) Infect. Immun. 69:5010-5015.
[66] Katial et al. (2002) Infect. Immun. 70:702-707.
[67] Covacci & Rappuoli (2000) J. Exp. Med. 19:587-592.
[68] WO93/18150.
[69] Covacci et al. (1993) Proc. Natl. Acad. Sci. USA 90: 5791-5795.
[70] Tummuru et al. (1994) Infect. Immun. 61:1799-1809.
[71] Marchetti et al. (1998) Vaccine 16:33-37.
[72] Telford et al. (1994) J. Exp. Med. 179:1653-1658.
[73] Evans et al. (1995) Gene 153:123-127.
[74] WO96/01272 & WO96/01273, especially SEQ ID NO:6.
[75] WO97/25429.
[76] WO98/04702.
[77] Costantino et al. (1992) Vaccine 10:691-698.
[78] Costantino et al. (1999) Vaccine 17:1251-1263.
[79] International patent application WO03/007985.
[80] Watson (2000) Pediatr Infect Dis J 19:331-332.
[81] Rubin (2000) Pediatr Clin North Am 47:269-285, v.
[82] Jedrzcjas (2001) Microbiol Mol Biol Rev 65:187-207.
[83] Bell (2000) Pediatr Infect Dis J 19:1187-1188.
[84] Iwarson (1995) APMIS 103:321-326.
[85] Gerlich et al. (1990) Vaccine 8 Suppl:S63-68 & 79-80.
[86] Gustafsson et al. (1996) N. Engl. J. Med. 334:349-355.
[87] Rappuoli et al. (1991) TIBTECH 9:232-238.
[88] Vaccines (1988) eds. Plotkin & Mortimer. ISBN 0-7216-1946-0.
[89] Del Guidice et al. (1998) Molecular Aspects of Medicine 19:1-70.
[90] Hsu et al. (1999) Clin Liver Dis 3:901-915.
[91] International patent application WO99/24578.
[92] International patent application WO99/36544.
[93] International patent application WO99/57280.
[94] International patent application WO02/079243.
[95] International patent application WO02/02606.
[96] Kalman et al. (1999) Nature Genetics 21:385-389.
[97] Read et al. (2000) Nucleic Acids Res 28:1397-406.
[98] Shirai et al. (2000) J. Infect. Dis. 181(Suppl 3):S524-S527.
[99] International patent application WO99/27105.
[100] International patent application WO00/27994.
[101] International patent application WO00/37494.
[102] International patent application WO99/28475.
[103] Ross et al. (2001) Vaccine 19:4135-4142.
[104] Sutter et al. (2000) Pediatr Clin North Am 47:287-308.
[105] Zimmerman & Spann (1999) Am Fam Physician 59:113-118, 125-126.
[106] Dreesen (1997) Vaccine 15 Suppl:S2-6.
[107] MMWR Morb Mortal Wkly Rep 1998 Jan 16;47(1):12, 19.
[108] Vaccines (1988) eds. Plotkin & Mortimer. ISBN 0-7216-1946-0.
[109] McMichael (2000) Vaccine 19 Suppl 1:S101-107.
[110] Schuchat (1999) Lancet 353(9146):51-6.
[111] International patent application WO02/34771.
[112] Dale (1999) Infect Dis Clin North Am 13:227-43, viii.
[113] Ferretti et al. (2001) PNAS USA 98: 4658-4663.
[114] Kuroda et al. (2001) Lancet 357(9264):1225-1240; see also pages 1218-1219.
[115] J Toxicol Clin Toxicol (2001) 39:85-100.
[116] Demicheli et al. (1998) Vaccine 16:880-884.
[117] Stepanov et al. (1996) J Biotechnol 44:155-160.
[118] Ingram (2001) Trends Neurosci 24:305-307.
[119] Rosenberg (2001) Nature 411:380-384.
[120] Moingeon (2001) Vaccine 19:1305-1326.
[121] EP-A-037250
[122] EP-A-0378881
[123] EP-A-0427347
[124] WO93/17712 .
[125] WO94/03208
[126] WO98/58668
[127] EP-A-0471177
[128] WO00/56360
[129] WO91/01146
[130] WO00/61761
[131]WO01/72337
[132] *Research Disclosure,* 453077 (Jan 2002)
[133] WO99/24578.
[134] WO99/36544.
[135] WO99/57280.
[136] WO00/22430.
[137] Tettelin et al. (2000) Science 287:1809-1815.
[138] WO96/29412.
[139] Pizza et al. (2000) Science 287:1816-1820.
[140] Current Protocols in Molecular Biology (F.M. Ausubel et al., eds., 1987) Supplement 30.
[141] Smith and Waterman, Adv. Appl. Math. (1981) 2: 482-489.
[142] WO03/009869.

## Claims

1. A composition comprising meningococcal outer membrane vesicles, an aluminium hydroxide adjuvant, a histidine buffer and sodium chloride, wherein: (a) the concentration of OMVs is less than 100µg/ml; and/or (b) the concentration of sodium chloride is greater than 7.5 mg/ml.

2. The composition of claim 1 wherein the concentration of sodium chloride is greater than 8 mg/ml,

3. The composition of claim 2 wherein the concentration of sodium chloride is 9 mg/ml.

4. The composition of any preceding claims wherein the concentration of OMVs is less than 75 µg/ml.

5. The composition of claim 4 wherein the concentration of OMVs is 50 µg/ml.

6. The composition of any one of the preceding claims wherein the histidine buffer is between pH 6.3 and pH 6.7.

7. The composition of claim 6 wherein the histidine buffer is pH 6.5.

8. The composition of any preceding claim wherein the adjuvant is present at 3.3 mg/ml (expressed as Al³⁺ concentration).

9. The composition of any one of the preceding claims for use as a medicament.

10. The use of a meningococcal outer membrane vesicle in the manufacture of a medicament for raising an immune response in a patient, wherein the medicament also comprises an aluminium hydroxide adjuvant, a histidine buffer and sodium chloride, and wherein: (a) the concentration of OMVs is less than 100µg/ml; and/or (b) the concentration of sodium chloride is greater than 7.5 mg/ml.

## Patentansprüche

1. Zusammensetzung, die Vesikel der äußeren Membranen (Outer Membrane Vesicles, OMVs) von Meningokokken, ein Aluminiumhydroxid-Adjuvans, einen Histidinpuffer und Natriumchlorid umfasst, worin: (a) die OMV-Konzentration weniger als 100 µg/ml beträgt und/oder (b) die Natriumchloridkonzentration mehr als 7,5 mg/ml beträgt.

2. Zusammensetzung nach Anspruch 1, worin die Natriumchloridkonzentration mehr als 8 mg/ml beträgt.

3. Zusammensetzung nach Anspruch 2, worin die Natriumchloridkonzentration 9 mg/ml beträgt.

4. Zusammensetzung nach einem der vorhergehenden Ansprüche, worin die OMV-Konzentration weniger als 75 µg/ml beträgt.

5. Zusammensetzung nach Anspruch 4, worin die OMV-Konzentration 50 µg/ml beträgt.

6. Zusammensetzung nach einem der vorhergehenden Ansprüche, worin der Histidinpuffer zwischen pH 6,3 und pH 6,7 ist.

7. Zusammensetzung nach Anspruch 6, worin der Histidinpuffer pH 6,5 ist.

8. Zusammensetzung nach einem der vorhergehenden Ansprüche, worin das Adjuvans in einer Konzentration von 3,3 mg/ml (ausgedrückt als Al³⁺-Konzentration) vorliegt.

9. Zusammensetzung nach einem der vorhergehenden Ansprüche zur Verwendung als Arzneimittel.

10. Verwendung eines Vesikels der äußeren Membran von Meningokokken in der Herstellung eines Arzneimittels zum Hervorrufen einer Immunreaktion bei einem Patienten, wobei das Arzneimittel auch ein Aluminiumhydroxid-Adjuvans, einen Histidinpuffer und Natriumchlorid umfasst und worin: (a) die OMV-Konzentration weniger als 100 µg/ml beträgt und/oder (b) die Natriumchloridkonzentration mehr als 7,5 mg/ml beträgt.

## Revendications

1. Composition comprenant des vésicules de membrane externe méningococciques, un adjuvant à base d'hydroxyde d'aluminium, un tampon histidine et du chlorure de sodium, dans laquelle : (a) la concentration en OMV est inférieure à 100 µg/ml ; et/ou (b) la concentration en chlorure de sodium est supérieure à 7,5 mg/ml.

2. Composition selon la revendication 1, dans laquelle la concentration en chlorure de sodium est supérieure à 8 mg/ml.

3. Composition selon la revendication 2, dans laquelle la concentration en chlorure de sodium est de 9 mg/ml.

4. Composition selon l'une quelconque des revendications précédentes, dans laquelle la concentration en OMV est inférieure à 75 µg/ml.

5. Composition selon la revendication 4, dans laquelle la concentration en OMV est de 50 µg/ml.

6. Composition selon l'une quelconque des revendications précédentes, dans laquelle le tampon histidine possède un pH compris entre pH 6,3 et pH 6,7.

7. Composition selon la revendication 6, dans laquelle le tampon histidine est à pH 6,5.

8. Composition selon l'une quelconque des revendications précédentes, dans laquelle l'adjuvant est présent selon une concentration de 3,3 mg/ml (exprimé en concentration en Al³⁺).

9. Composition selon l'une quelconque des revendications précédentes, pour une utilisation comme médicament.

10. Utilisation d'une vésicule de membrane externe méningococcique dans la préparation d'un médicament destiné à induire une réponse immunitaire chez un patient, dans laquelle le médicament comprend également un adjuvant à base d'hydroxyde d'aluminium, un tampon histidine et du chlorure de sodium, et dans laquelle : (a) la concentration en OMV est inférieure à 100 µg/ml ; et/ou (b) la concentration en chlorure de sodium est supérieure à 7,5 mg/ml.
